# EUROPÄISCHE PATENTSCHRIFT

(11) **EP 1 424 937 B1**
(45) Veröffentlichungstag und Bekanntmachung des Hinweises auf die Patenterteilung: **17.11.2004**
(21) Anmeldenummer: 02791457.1
(22) Anmeldetag: 17.07.2002
(51) Int. Cl.: A61B 5/07, A61B 5/00

(54) **MEDIZINISCHES IMPLANTATSYSTEM**
MEDICAL IMPLANT SYSTEM
SYSTEME D'IMPLANT MEDICAL

(30) Priorität: 28.07.2001 DE 10137011
(43) Veröffentlichungstag der Anmeldung: 09.06.2004
(73) Patentinhaber: Aesculap AG & Co. KG, 78532 Tuttlingen (DE)
(72) Erfinder: GRUPP, Thomas, 73072 Donzdorf (DE); KOZAK, Josef, 78532 Tuttlingen (DE)
(74) Vertreter: Böhme, Ulrich, Dr. Dipl.-Phys.
(86) Internationale Anmeldenummer: PCT/EP2002/007927
(87) Internationale Veröffentlichungsnummer: WO 2003/011133

(56) Entgegenhaltungen:
- WO-A-01/22880
- WO-A-96/17223
- WO-A-99/45352
- DE-C- 3 914 164
- US-A- 5 792 076
- US-A- 6 005 242
- JIE L ET AL: "Sol-gel glass as a matrix for chemical and biochemical sensing" TRAC, TRENDS IN ANALYTICAL CHEMISTRY, ANALYTICAL CHEMISTRY. CAMBRIDGE, GB, Bd. 16, Nr. 4, 1. April 1997 (1997-04-01), Seiten 200-211, XP004064451 ISSN: 0165-9936

## Beschreibung

Die Erfindung betrifft ein medizinisches Implantatsystem mit einem Implantat aus einem Verbundwerkstoff, in welchen Glasfasern eingebettet sind, wobei ein in das Implantat eingebettetes, mindestens eine der Glasfasern umfassendes Sensorelement mit einer Meßeinrichtung verbunden ist, die eine physikalische Eigenschafts des Sensorelements oder dessen Umgebung oder deren Änderung bestimmt.

Medizinische Implantate, beispielsweise Knochenplatten, Marknägel, Endoprothesen, Osteosynthesesysteme für die Wirbelsäule etc. werden üblicherweise aus metallischen Werkstoffen hergestellt, es sind aber auch Implantate bekannt, die aus einem Verbundwerkstoff bestehen, in welchen zur Verstärkung Glasfasern eingebettet sind, insbesondere bestehen derartige medizinische Implantate aus sterilisierbaren, ausgesuchten Kunststoffen wie Polyetheretherketon, Polyamiden etc.

Wenn diese Implantate in den Körper eingesetzt sind, sind sie unterschiedlichen Einflüssen ausgesetzt, beispielsweise unterschiedlichen Dehnungen und Spannungen, Temperaturentwicklungen oder chemischen Umgebungen. Es wäre für den behandelnden Art von Interesse, diese unterschiedlichen Parameter zu erfahren, da sie Auskunft geben über den Heilungsverlauf oder über möglicherweise auftretende Probleme.

In der US-A-5 792 076 sind medizinische Vorrichtungen beschrieben, in die Glasfasern eingebettet werden können, um über die Glasfasern bestimmte physikalische Eigenschaften von Implantaten oder medizinischen Vorrichtungen zu erfassen. Im Einzelfall wird dabei eine einzelne Faser eingebettet, es ist auch beschrieben, daß mehrere Fasern eingebettet sind, beispielsweise sechs Fasern. Diese Fasern werden beispielsweise längs des Umfanges eines Stabes in regelmäßigen Mustern angeordnet, beispielsweise in Umfangsrichtung oder in radialer Richtung in gleichem Abstand. In all diesen Fällen werden die Glasfasern also ausschließlich als Sensoren verwendet.

Ausgehend von diesem Stand der Technik liegt dem Anmeldegegenstand die Aufgabe zugrunde, ein gattungsgemäßes medizinisches Implantatsystem so zu verbessern, daß man Informationen über physikalische Eigenschaften im Implantat und in seiner Umgebung erhalten kann.

Diese Aufgabe wird bei einem medizinischen Implantat der eingangs beschriebenen Art erfindungsgemäß dadurch gelöst, daß die Glasfasern in Form eines Gewebes, eines Gewirkes oder eines Vlieses als mechanische Verstärkung in den Verbundwerkstoff eingebettet sind.

Die Glasfasern bilden also ein Netzwerk aus, das insgesamt in den Verbundwerkstoff eingebettet ist und diesen dadurch verstärkt.

Glasfasern sind zwar in verschiedener Form als Material zur Verstärkung bekannt (DE 39 14 164 C1), jedoch dienen die Glasfasern dabei ausschließlich als Verstärkungsmaterial. Im Rahmen der vorliegenden Erfindung werden die Glasfasern jedoch gleichzeitig als Verstärkung und als Sensorelement eingesetzt, und dies ist aus dem bekannten Stand der Technik nicht naheliegend gewesen.

Dabei werden unter dem Begriff "Glasfaser" alle faserförmigen, in den Verbundwerkstoff einbettbaren Substanzen verstanden, die in der Lage sind elektromagnetische Strahlung zu führen und zu übertragen, vorzugsweise bestehen diese Fasern aus Quarzglas, es können aber auch andere Substanzen Verwendung finden, beispielsweise Fasern aus Kunststoff, sogenannte Plastic Optical Fibres (POF).

Je nach den mechanischen Anforderungen können die Glasfasern dabei in bestimmten Bereichen des Implantates konzentriert oder aber über die gesamte Ausdehnung des Implantates verteilt sein.

Vorzugsweise ist die Meßeinrichtung so ausgebildet, daß sie elektromagnetische Strahlung in das Sensorelement einspeist und aus der Art der durchgehenden und/oder reflektierten Strahlung physikalische Eigenschaften des Sensorelementes oder von dessen Umgebung bestimmt.

Die Glasfaser des Sensorelementes ist gemäß einer bevorzugten Ausführungsform mit einer strahlungsreflektierenden Beschichtung versehen.

Bei einer ersten bevorzugten Ausführungsform besteht das Sensorelement im wesentlichen aus der eine Sensorfaser ausbildenden Glasfaser. Bei dieser Ausführungsform ist also die in den Verbundwerkstoff eingebettete Glasfaser gleichzeitig Sensor und Übertragungselement für die elektromagnetische Strahlung.

Es sind eine größere Anzahl von unterschiedlichen Ausgestaltungen möglich, bei denen die Glasfaser als Sensorfaser wirkt, beispielsweise kann in die Sensorfaser mindestens ein als Bragg-Gitter wirkender Bereich eingearbeitet sein. In einem solchen Bereich, der periodische Änderungen des Brechungsindex in Längsrichtung der Sensorfaser aufweist, wird Strahlung reflektiert, die sich bei der Reflexion überlagert und sich nur für ganz bestimmte Wellenlängen in Rückrichtung verstärkt. Diese Wellenlänge hängt von der Periodizität des Bragg-Gitterbereiches ab und ändert sich mit dieser Periodizität. Jede Längenänderung der Sensorfaser oder jede Änderung der Periodizität des Bragg-Gitters, die aufgrund von äußeren Einflüssen eintritt, kann auf diese Weise in Form einer Wellenlängenverschiebung festgestellt werden.

Bei einer anderen bevorzugten Ausführungsform kann vorgesehen sein, daß in die Sensorfaser eine durch die eingespeiste elektromagnetische Strahlung zu Fluoreszenz angeregte Substanz eingebettet ist, deren Fluoreszenzeigenschaften unter Einwirkung der Umgebung außerhalb der Sensorfaser Änderungen erfahren. Diese Änderungen können mechanische Änderungen sein, insbesondere kann jedoch die Fluoreszenzeigenschaft der eingebetteten Substanz durch die chemische Umgebung der Sensorfaser beeinflußt werden, beispielsweise kann die Fluoreszenz durch bestimmte Substanzen in der Umgebung gelöscht werden.

Bei einer weiteren bevorzugten Ausführungsform ist vorgesehen, daß die strahlungsreflektierende Beschichtung aus einer Substanz besteht, die unter Einwirkung der Umgebung außerhalb der Sensorfaser das Reflexionsverhalten für die elektromagnetische Strahlung in der Sensorfaser verändert. Dadurch wird die durch die Sensorfaser hindurchtretende und reflektierte Strahlungsmenge verändert, und dies läßt sich feststellen.

Jede Änderung der Eigenschaften in der Strahlung kann detektiert werden, es kann sich dabei um Änderungen der Wellenlänge, der Phasenlage, der Polarisation etc. handeln, wesentlich ist lediglich, daß diese Änderungen in klar erkennbarem Zusammenhang mit Änderungen der Eigenschaften in der Umgebung der Sensorfaser stehen, also beispielsweise mit Änderungen der mechanischen Spannung, der Temperatur oder der stofflichen Zusammensetzung.

Bei einer weiteren bevorzugten Ausführungsform kann vorgesehen sein, daß das Sensorelement die Glasfaser umfaßt und ein weiteres Sensorglied, welches über die Glasfaser mit der Meßeinrichtung verbunden ist. Bei dieser Ausgestaltung wirkt die Glasfaser im wesentlichen als Übertragungselement zwischen dem Sensorglied und der Meßeinrichtung.

Beispielsweise kann das Sensorglied ein Drucksensor mit einer flexiblen Membran und einem von dieser bewegbaren Spiegelelement sein, welches die in die Glasfaser eingespeiste elektromagnetische Strahlung je nach Stellung unterschiedlich reflektiert.

Bei einer weiteren Ausführungsform kann das Sensorglied ein Fabry-Pérot-Interferometer sein.

Beispielsweise kann dabei vorgesehen sein, daß das Fabry-Perot-Interferometer als auf das Ende der Glasfaser aufkontaktiertes Dünnschicht-Interferometer ausgebildet ist, dessen aktive Schicht unter dem Einfluß der Umgebung Dimensionsänderungen erfährt. Eine solche aktive Schicht kann beispielsweise porös ausgebildet sein und quellen, wenn sie mit einer Flüssigkeit in Verbindung kommt, auf diese Weise ist zum Beispiel feststellbar, ob ein Implantat noch abgedichtet ist oder eine erwünschte oder unerwünschte Öffnung zur Umgebung aufweist.

Bei einer anderen Ausführungsform ist vorgesehen, daß das Fabry-Perot-Interferometer zwei Glasfasern mit polierten Endflächen umfaßt, deren Abstand durch Umgebungseinflüsse veränderbar ist. Diese Ausgestaltung ist insbesondere dann günstig, wenn Dehnungen oder Verschiebungen innerhalb eines Implantates festgestellt werden sollen.

Die Glasfaser des Sensorelementes kann direkt mit der Meßeinrichtung verbunden sein, wobei die Meßeinrichtung im Innern des Körpers getragen werden kann, aber auch außerhalb. Im letzteren Fall wird die Glasfaser aus dem Implantat durch das Körpergewebe nach außen geführt, so daß dort eine Verbindung zu der Meßeinrichtung hergestellt werden kann.

Besonders günstig ist es, wenn die Meßeinrichtung ein in den Körper implantierbarer Mikrocontroller ist.

Bei einer besonders bevorzugten Ausführungsform ist die Glasfaser mit einem Übertrager verbunden, der ohne körperliche Verbindung Signale mit der Meßeinrichtung austauscht.

Dieser Übertrager kann insbesondere in den Körper implantierbar sein, beispielsweise kann es sich dabei um einen Transponder handeln.

Bei einer besonders günstigen Ausführungsform ist der Übertrager eine Lichtquelle, der ein Lichtempfänger zugeordnet ist. Es hat sich herausgestellt, daß Licht unterschiedlicher Wellenlänge Körpergewebe in gewissem Umfange durchdringen kann, so daß zwischen einem Lichtempfänger und einer Lichtquelle, von denen ein Teil im Körper und ein Teil außerhalb angeordnet sind, durch Licht eine Übertragung von Strahlungsenergie möglich ist, insbesondere dann, wenn die Lichtquelle elektromagnetische Strahlung im Bereich zwischen 650 und 1000 nm aussendet.

Bei einer besonders bevorzugten Ausführungsform ist der Meßeinrichtung ein Strahlungssender zugeordnet, der über eine Glasfaser im Implantat Strahlung in das Innere des Implantates transportiert. Ein solcher Strahlungssender kann dazu verwendet werden, zusätzlich zur Bestimmung der physikalischen Eigenschaften des Implantates durch die eingekoppelte Strahlung auf das Implantat einzuwirken und dieses zu verändern, beispielsweise durch Erwärmung in bestimmten Bereichen oder dergleichen.

Es kann dabei vorgesehen sein, daß der Transport der Strahlung über eine Glasfaser erfolgt, die zusätzlich zu der Glasfaser eines Sensorelementes in das Implantat eingebettet ist, es kann aber auch vorgesehen sein, daß der Transport der Strahlung über die Glasfaser eines Sensorelementes erfolgt. In diesem Fall ist es vorteilhaft, wenn entsprechende Schaltelemente Verwendung finden, welche die Glasfaser wahlweise mit der Meßeinrichtung und mit dem Strahlungssender verbinden.

Besonders vorteilhaft ist eine Ausgestaltung, bei der Wellenlänge und Intensität der transportierten Strahlung so gewählt sind, daß die Strahlung in dem Verbundwerkstoff des Implantates mechanische und/oder stoffliche Veränderungen hervorruft. Beispielsweise ist es dadurch möglich, eine zusätzliche Aushärtung eines polymeren Verbundwerkstoffes in bestimmten Bereichen vorzunehmen oder umgekehrt eine Schwächung durch Zerstörung des Verbundwerkstoffes, so daß auf diese Weise die mechanischen Eigenschaften des Implantates in größeren Bereichen oder aber auch lokal geändert werden können.

Bei einer besonders bevorzugten Ausführungsform ist dabei vorgesehen, daß der Meßeinrichtung und dem Strahlungssender eine Steuerung zugeordnet ist, die den Strahlungssender in Abhängigkeit von den Meßgrößen der Meßeinrichtung aktiviert. Bei dieser Ausgestaltung ist es möglich, die physikalischen Daten des Implantates laufend zu bestimmen, beispielsweise die auf das Implantat übertragenen mechanischen Spannungen, die zum Beispiel ein Maß für den Heilungsprozeß sind, diese Spannungen nehmen mit zunehmender Stabilität an der Knochenverbindung ab, da ein Teil der Belastungen durch den Knochen übernommen wird. Es ist dann günstig, entsprechend dieser Regeneration der Knochenverbindung die Festigkeit des Implantates herabzusetzen, so daß die Kraftübertragungsfunktion zunehmend von dem heilenden Knochen übernommen wird.

Die nachfolgende Beschreibung bevorzugter Ausführungsformen der Erfindung dient im Zusammenhang mit der Zeichnung der näheren Erläuterung. Es zeigen:
- Figur 1:: eine schematische Ansicht eines Implantats in Form einer Knochenplatte mit einer drahtlosen Verbindung zu einer Meßeinrichtung;
- Figur 2:: eine schematische Ansicht eines plattenförmigen Implantates mit einer netzförmigen Glasfaserverstärkung;
- Figur 3:: eine schematische Ansicht eines Implantats in Form einer Knochenplatte mit einer an mehrere Glasfasern angeschlossenen Meßeinrichtung und mit einer Strahlungsquelle zur Einführung von Strahlung in eine nicht mit der Meßeinrichtung verbundene Glasfaser;
- Figur 4:: eine Ansicht ähnlich Figur 3 mit einer Schalteinrichtung zur wahlweisen Verbindung von Glasfasern im Implantat mit der Meßeinrichtung oder mit der Strahlungsquelle;
- Figur 5:: eine schematische Seitenansicht einer Glasfaser mit Bragg-Gitter-Bereichen unterschiedlicher Periodizität;
- Figur 6:: eine schematische Seitenansicht einer Glasfaser mit eingebetteten fluoreszierenden Farbstoffpartikeln;
- Figur 7:: eine schematische Seitenansicht einer Glasfaser mit einer Ummantelung mit veränderlichen Transmissionseigenschaften;
- Figur 8:: eine schematische Seitenansicht eines mit einer Glasfaser verbundenen Fabry-Pérot-Interferometers mit zwei gegeneinander bewegten Glasfaserstükken;
- Figur 9:: eine Ansicht ähnlich Figur 8 mit einer dimensionsveränderlichen aktiven Schicht und
- Figur 10:: eine schematische Seitenansicht einer Glasfaser mit einem Membrandrucksensor.

Die Erfindung wird nachfolgend am Beispiel einer Knochenplatte erläutert, es versteht sich aber, daß die Erfindung allgemein für in den Körper einsetzbare medizinische Implantate verwendbar ist und nicht auf Knochenplatten beschränkt ist.

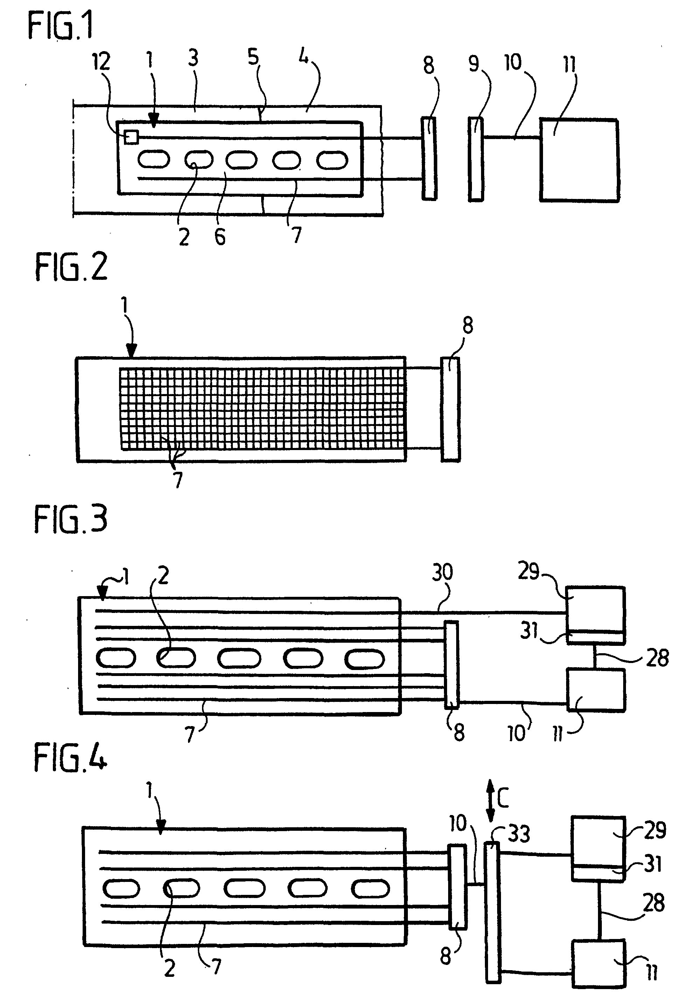

Ein Implantat 1 in Form einer Knochenplatte mit Öffnungen 2 zur Aufnahme von Knochenschrauben ist in an sich bekannter Weise mittels Knochenschrauben so mit zwei Knochenfragmenten 3, 4 verbunden, daß diese in einer bestimmten Relativposition zueinander fixiert sind, so daß beispielsweise eine Bruchstelle 5 verheilen kann (Figur 1). Das Implantat 1 besteht aus einem Kunststoffmaterial, beispielsweise aus einem resorbierbaren Kunststoff wie Polylactid (PLLA, PL DLLA), Polyglycolit (PGA) oder Trimethylencarbonat (TMC), und in dieses Kunststoffmaterial 6 sind Glasfasern 7 eingebettet. Im Ausführungsbeispiel der Figur 1 sind schematisch nur zwei einzelne Glasfasern 7 dargestellt, die sich in Längsrichtung des plattenförmigen Implantates 1 erstrecken, im Ausführungsbeispiel der Figur 2 sind eine Vielzahl von Glasfasern 7 in Form eines Netzes angedeutet, welches insgesamt in das Kunststoffmaterial 6 eingebettet ist, hier sind die unterschiedlichsten Anordnungen und Konzentrationen von Glasfasern in dem Kunststoffmaterial 6 möglich. Die Glasfasern verstärken durch diese Einbettung das Kunststoffmaterial 6, und dementsprechend werden unterschiedliche Verteilungen im Implantat gewählt, je nach den mechanischen Festigkeitsanforderungen.

Die Glasfasern 7 im Ausführungsbeispiel der Figur 1 sind mit einem Übertragungselement 8 verbunden, beispielsweise einem üblichen Transponder, der am Implantat 1 selbst oder im Abstand vom Implantat 1 im Innern des Körpers des Patienten oder aber auch auf der Oberfläche des Körpers des Patienten angeordnet werden kann, es kann sich dabei auch um ein optisches Element handeln, welches Licht empfangen und aussenden kann, beispielsweise ein kleiner Parabolspiegel, eine Linse oder dergleichen. Im Ausführungsbeispiel der Figur 1 sind alle im Implantat 1 angeordneten Glasfasern 7 mit dem Übertragungselement 8 verbunden, im Ausführungsbeispiel der Figur 2 nur einige, während andere Glasfasern ausschließlich der Verstärkung des Implantates 1 dienen. Dies kann von Fall zu Fall unterschiedlich gewählt werden, im Extremfall genügt es, eine einzige Glasfaser 7 im Implantat 1 mit einem solchen Übertragungselement 8 zu verbinden.

Dem Übertragungselement 8 ist ein entsprechendes Übertragungselement 9 zugeordnet, welches über eine Leitung 10 mit einer Meßeinrichtung 11 verbunden ist. Zwischen den Übertragungselementen 8 und 9 können Signale ausgetauscht werden, es kann sich dabei um elektrische Signale, um optische Signale, um mechanische Signale (Ultraschall) handeln, wesentlich ist lediglich, daß von dem Übertragungselement 8 in die Glasfaser und gegebenenfalls von der Glasfaser in das Übertragungselement 8 elektromagnetische Energie übertragen wird, die im Übertragungselement 8 in Signale umgesetzt wird, die dann in beliebiger Weise zum Übertragungselement 9 und damit zur Meßeinrichtung 11 geleitet werden können. Insbesondere können die Übertragungselement 8 und 9 bei einer Anordnung des Übertragungselements 8 im Innern des Körpers zwischen sich eine elektromagnetische Strahlung mit einer Wellenlänge zwischen 650 und 1000 Nanometer austauschen, diese elektromagnetische Strahlung kann das Körpergewebe bis zu einer bestimmten Tiefe durchdringen und kann somit eine Signalverbindung zwischen den beiden Übertragungselementen 8 und 9 herstellen, und zwar sowohl in Einstrahlrichtung als auch in Ausstrahlrichtung.

Die auf diese Weise in die Glasfaser 7 eingekoppelte Strahlung wird in der Glasfaser 7 geführt und durch diese selbst oder durch ein mit ihr verbundenes Sensorglied 12 verändert, und zwar abhängig von den physikalischen Zustandsdaten der Glasfaser 7, des Sensorgliedes 12 oder der Umgebung derselben. Die daraufhin aus der Glasfaser 7 dem Übertragungselement 8 in Rückrichtung zugeführte Strahlung ist dementsprechend verändert, und diese Veränderung läßt sich von der Meßeinrichtung 11 feststellen, die damit eine Rückmeldung über Änderungen des physikalischen Zustands der Glasfaser, des Sensorgliedes 12 und/oder der Umgebung derselben erhält.

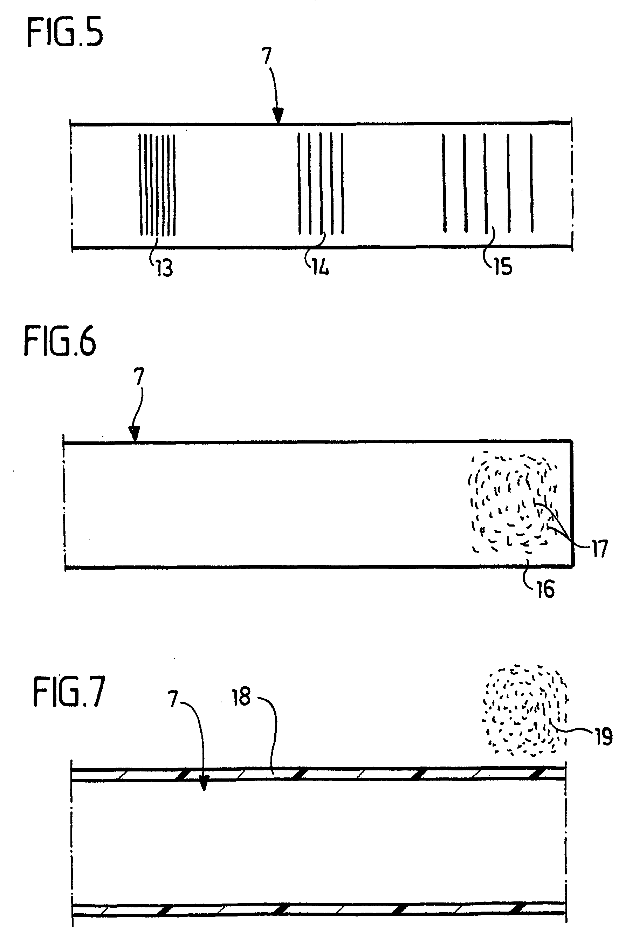

Die Möglichkeiten zur Einwirkung auf die in die Glasfaser 7 eingespeiste elektromagnetische Strahlung sind vielfältig, es lassen sich auf diese Weise Längenänderungen, Verformungen, mechanische Zugspannungen, Kräfte, Schwingungen, Drücke, Drehwinkel, elektrische oder magnetische Feldstärken, Ströme, Temperaturen, Feuchte, ionisierende Strahlungen oder Konzentration oder Anwesenheit von chemischen Substanzen bestimmen, dies ist lediglich eine Auswahl der möglichen physikalischen Zustände, die auf diese Weise feststellbar sind. Anhand der Figuren 5 bis 10 werden nachstehend einige Beispiele der Beeinflussung der elektromagnetischen Strahlung in einer Glasfaser erörtert.

In Figur 5 ist ein Ausschnitt einer Glasfaser 7 dargestellt, in dieser Glasfaser sind in Längsrichtung im Abstand voneinander angeordnet verschiedene Bereiche 13, 14, 15 vorgesehen, bei denen in Längsrichtung der Faser periodische Änderungen des Brechungsindex auftreten. Diese lassen sich zum Beispiel dadurch erzeugen, daß eine beispielsweise mit Germaniumdioxid dotierte Quarzglasfaser über eine mikrolithographische Maske mit Ultraviolettlicht von 240 nm Wellenlänge bestrahlt wird. Es entsteht dadurch in jedem Bereich 13, 14, 15 eine Anordnung eines Bragg-Gitters, wobei die Periodizität und damit die Gitterkonstante in verschiedenen Bereichen 13, 14, 15 unterschiedlich gewählt werden.

An jedem dieser Bragg-Gitter wird durch Interferenzstrahlung eine ganz bestimmte Wellenlänge reflektiert, diese Wellenlänge ist abhängig von der Periodizität des Gitters und ändert sich damit auch, wenn dieses die Periodizität ändert. Eine solche Änderung der Periodizität oder Gitterkonstante kann durch äußere Einflüsse erfolgen, beispielsweise durch Dehnung der Glasfaser, durch Biegung der Glasfaser, durch Erwärmung etc. Da in jedem Bereich 13, 14, 15 nur Strahlung einer bestimmten Wellenlänge reflektiert wird, kann man an der Wellenlänge der reflektierten Strahlung sofort ablesen, an welchem Bereich eine Reflexion erfolgt ist, außerdem gibt die Verschiebung der Wellenlänge Auskunft über Änderungen der Gitterabstände in diesen Bereichen, also zum Beispiel über die Dehnung der Glasfaser in bestimmten Bereichen. Diese kann in den Bereichen 13, 14, 15 unterschiedlich sein, die Meßeinrichtung kann aus der reflektierten Strahlung Aussagen darüber machen, wie groß eine Dehnung in jedem der Bereiche 13, 14, 15 ist. Damit erhält man insbesondere bei der Verwendung von mehreren derartigen Glasfasern eine genaue Auskunft über die Verformung des Implantates 1 im Körper und damit zum Beispiel über den Heilungsfortgang beim Zusammenwachsen von Knochenfragmenten. Die Dehnung aufgrund der ausgeübten Kräfte wird am größten sein, wenn die Knochenfragmente noch nicht zusammengewachsen sind, und sie wird mit dem Heilungsfortgang laufend abnehmen.

Bei dem Ausführungsbeispiel der Figur 6 sind in die Glasfaser 7 in einem bestimmten Bereich 16 Farbstoffpartikel 17 eingebettet, die durch in die Glasfaser 7 eintretende elektromagnetische Strahlung zur Fluoreszenz angeregt werden. Die auf diese Weise abgegebene Strahlung kann von der Meßeinrichtung bestimmt werden. Umgebungseinflüsse, beispielsweise bestimmte chemische Substanzen in der Umgebung des Bereiches 16, können die Fluoreszenz beeinflussen, beispielsweise kann die Fluoreszenzintensität herabgesetzt oder aber die Fluoreszenz ganz gelöscht werden. Die Meßeinrichtung erhält auf diese Weise Information über die Anwesenheit bestimmter chemischer Substanzen in der Umgebung des Bereiches 16.

Beim Ausführungsbeispiel der Figur 7 ist die Glasfaser 7 mit einer Beschichtung 18 umhüllt, die einen Austritt der durch die Glasfaser 7 geführten elektromagnetischen Strahlung verhindert. Diese Beschichtung kann mit chemischen Stoffen 19 in der Umgebung reagieren und sich dabei so umsetzen, daß die Austrittseigenschaften der elektromagnetischen Strahlung in dem Bereich geändert werden, in dem sich der chemische Stoff 19 befindet, und auf diese Weise erhält man wieder eine Änderung der reflektierten Strahlung in Abhängigkeit von bestimmten chemischen Stoffen 19 in der Umgebung der Glasfaser 7.

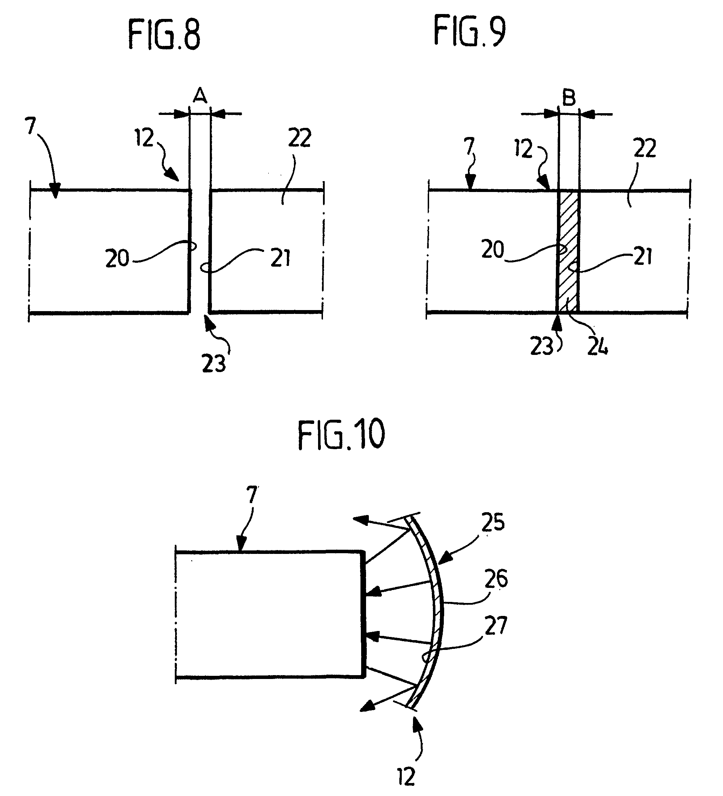

Beim Ausführungsbeispiel der Figur 8 steht das plangeschliffene Ende 20 der Glasfaser 7 einem ebenfalls plangeschliffenen Ende 21 eines Glasfaserstückes 22 gegenüber, wobei zwischen den Enden 20 und 21 ein sehr schmaler Spalt 23 entsteht, die Spaltbreite A kann beispielsweise in der Größenordnung von 50 mm liegen. Diese Anordnung bildet ein Fabry-Pérot-Interferometer aus und reflektiert Strahlung einer ganz bestimmten Wellenlänge, diese ist abhängig von der Spaltbreite A. Verschieben sich die beiden Enden 20 und 21 relativ zueinander, ergibt sich also auch eine Verschiebung der Wellenlänge der reflektierten Strahlung, und dies läßt sich sehr empfindlich feststellen. Auch auf diese Weise lassen sich zum Beispiel Dehnungen des Implantates, die auf die Glasfaser 7 und das Glasfaserstück 22 übertragen werden, ohne weiteres feststellen.

Beim Ausführungsbeispiel der Figur 9 ist eine ähnliche Anordnung gewählt, jedoch ist in den Spalt 23 eine aktive Lage 24 eingesetzt, die ihre Dimension, beispielsweise ihr Volumen, in Abhängigkeit von Umgebungseinflüssen ändert. Es kann sich dabei beispielsweise um eine poröse Struktur handeln, die beim Eintritt von Flüssigkeit in die Poren aufquillt. Die Spaltbreite B verändert sich dadurch, und dies führt zu einer Veränderung der Wellenlänge der an der Fabry-Pérot-Anordnung reflektierten Strahlung.

Die Fabry-Pérot-Anordnungen der Figuren 8 und 9 bilden somit ein Sensorglied 12 aus, das über die Glasfaser 7 mit der Meßeinrichtung 11 in Verbindung steht, bei den Ausführungsbeispielen der Figuren 5 bis 7 dagegen ist die Glasfaser 7 selbst ein Sensorelement, es handelt sich hier also um Glasfasern, die selbst Sensorfasern sind.

Bei dem Ausführungsbeispiel der Figur 10 ist der Glasfaser 7 ein Sensorglied 12 in Form eines Drucksensors 25 zugeordnet. Dieser umfaßt eine flexible Membran 26, die einseitig mit einer Spiegelschicht 27 versehen ist. Ordnet man diesen Drucksensor 25 am Ende einer Glasfaser 7 an, so ändert sich mit der Verformung der Membran 26, die druckabhängig erfolgt, die in die Glasfaser 7 zurückgeworfene elektromagnetische Strahlung, und damit erhält man wieder ein Maß für den Druck am Ende der Glasfaser 7.

Bei dem Ausführungsbeispiel der Figuren 1 und 2 sind Glasfasern 7, die aus dem Implantat 1 herausgeführt sind, direkt oder indirekt mit der Meßeinrichtung 11 verbunden.

Dies ist bei der Ausführung gemäß Figur 3, die ähnlich aufgebaut ist wie die der Figur 1 und bei der gleiche Teile entsprechende Bezugszeichen tragen, ähnlich gelöst, die Verbindung des Übertragungselementes 8 mit der Meßeinrichtung 11 ist bei dem Ausführungsbeispiel in der Figur 3 durch eine Leitung 10 symbolisiert, es kann sich dabei um eine körperliche Leitung oder um eine leitungslose Übertragungsstrecke handeln.

Zusätzlich ist bei dieser Ausführungsform eine Strahlungsquelle 29 vorgesehen, die mit einer oder mehreren Glasfasern 30 in Verbindung stehen, die in das Kunststoffmaterial 6 des Implantates 1 eingebettet sind. Im Ausführungsbeispiel der Figur 3 ist nur eine derartige Glasfaser 30 dargestellt, die direkt mit der Strahlungsquelle 29 verbunden ist, dies ist lediglich als schematische Darstellung aufzufassen. Auch hier können mehrere Glasfasern 30 vorgesehen sein, die in ähnlicher Weise, wie die Glasfasern 7 mit der Meßeinrichtung verbunden sind, ihrerseits mit der Strahlungsquelle 29 verbunden sind, also über Übertragungselemente, die im Körper oder außerhalb angeordnet sein könnten, etc.

Die Strahlungsquelle 29 kann in die Glasfasern 30 eine elektromagnetische Strahlung einspeisen, die im Innern des Implantates 1 austritt und dort eine direkte Beeinflussung der Umgebung erzeugt, beispielsweise eine Aufwärmung des umgebenden Kunststoffmaterials 6 oder aber eine zusätzliche Aushärtung durch erhöhte Polymerisation oder aber eine Auflösung von Polymerisationsverbindungen etc. Hier sind eine Vielzahl von Wirkungen denkbar, die abhängen von der Natur des verwendeten Kunststoffmaterials 6 und von der Natur der eingespeisten elektromagnetischen Strahlung. Die Wirkung dieser eingespeisten elektromagnetischen Strahlung ist in jedem Falle eine Beeinflussung der physikalischen Daten des Kunststoffmaterials 6 und eventuell der Umgebung des Implantates 1, beispielsweise kann die Festigkeit des Implantates lokal oder flächendeckend erhöht oder erniedrigt werden. Den Ort der Einwirkung kann man durch entsprechende Anordnung der Glasfasern 30 im Implantat 1 bestimmen, die Art der Einwirkung durch eine entsprechende Auswahl einer bestimmten Strahlung.

Die Strahlungsquelle 29 kann völlig unabhängig von der Meßeinrichtung 13 aktiviert werden, es ist aber besonders vorteilhaft, wenn, wie in Figur 3 dargestellt, der Strahlungsquelle 29 eine Steuerung 31 zugeordnet ist, die die Strahlungsquelle 29 in Abhängigkeit von den Meßdaten der Meßeinrichtung 11 ein- und ausschaltet. Zu diesem Zweck ist die Meßeinrichtung 11 über eine Leitung 28 mit der Steuerung 31 verbunden.

Stellt beispielsweise die Meßeinrichtung 11 fest, daß die Dehnung des Implantates 1 in einem bestimmten Bereich abnimmt, so ist dies ein Zeichen dafür, daß ein Teil der Kraftübertragung durch verheilende Knochenfragmente übernommen worden ist, es kann dann durch Einspeisen von elektromagnetischer Strahlung in Glasfasern 30 die Festigkeit des Implantates 1 durch Auflösen eines Teils des Kunststoffmaterials 6 herabgesetzt werden, so daß die Stützfunktion des Implantates 1 entsprechend der Zunahme der Stabilität der Knochenverbindung abnimmt. Damit ist eine optimale Anpassung dieser Größen aneinander möglich, außerdem ist es für die Heilung förderlich, wenn die Knochenverbindung entsprechend dem Heilvorgang zunehmend belastet wird.

Bei dem Ausführungsbeispiel der Figur 3 erfolgt die Einführung der von der Strahlungsquelle 29 erzeugten Strahlung über Glasfasern 30, die von den Glasfasern 7 der Meßeinrichtung verschieden sind.

Es ist auch möglich, sowohl die Messung der physikalischen Zustandsdaten als auch die Einspeisung von elektromagnetischer Strahlung über dieselben Glasfasern 7 vorzunehmen, dies ist in Figur 4 schematisch dargestellt. Zu diesem Zweck ist zwischen das Übertragungselement 8 einerseits und die Meßeinrichtung 11 und die Strahlungsquelle 29 andererseits ein optischer Schalter 33 eingeschaltet, der wahlweise eine Verbindung der Glasfasern 7 mit der Meßeinrichtung 11 oder der Strahlungsquelle 29 ermöglicht. In Figur 4 ist dies durch den Doppelpfeil C symbolisch angedeutet. Schalter dieser Art stehen in verschiedener Weise zur Verfügung, es kann sich dabei um mechanische Schalter handeln, die beispielsweise ein Glasfaser zwischen zwei Einkoppelstellen verschieben, oder aber auch um Schalter, die elektromagnetisch, piezoelektrisch oder thermisch arbeiten, hier sind dem Fachmann eine große Anzahl unterschiedlicher Schalter bekannt, die zu diesem Zweck eingesetzt werden können.

Der optische Schalter 33 kann gegebenenfalls auch automatisch betätigt werden, so daß sichergestellt ist, daß beispielsweise abwechselnd über die Glasfaser 7 eine Messung des physikalischen Zusandes vorgenommen wird und Strahlungsenergie zur Beeinflussung der Glasfaserumgebung eingestrahlt wird.

## Patentansprüche

1. Medizinisches Implantatsystem mit einem Implantat (1) aus einem Verbundwerkstoff, in welchen Glasfasern (7) eingebettet sind, wobei ein in das Implantat (1) eingebettetes, mindestens eine der Glasfasern (7) umfassendes Sensorelement mit einer Meßeinrichtung (11) verbunden ist, die eine physikalische Eigenschafts des Sensorelements oder dessen Umgebung oder deren Änderung bestimmt, **dadurch gekennzeichnet, daß** die Glasfasern (7) in Form eines Gewebes, eines Gewirkes oder eines Vlieses als mechanische Verstärkung in den Verbundwerkstoff eingebettet sind.

2. Implantatsystem nach Anspruch 1, **dadurch gekennzeichnet, daß** die Glasfasern (7) im Verbundwerkstoff über die gesamte Ausdehnung des Implantates (1) verteilt sind.

3. Implantatsystem nach einem der voranstehenden Ansprüche, **dadurch gekennzeichnet, daß** die Meßeinrichtung (11) elektromagnetische Strahlung in das Sensorelement einspeist und aus der Art der durchgehenden und/oder reflektierenden Strahlung physikalische Eigenschaften des Sensorelementes oder von dessen Umgebung bestimmt.

4. Implantatsystem nach Anspruch 3, **dadurch gekennzeichnet, daß** die Glasfaser (7) des Sensorelementes mit einer strahlungsreflektierenden Beschichtung (18) versehen ist.

5. Implantatsystem nach einem der voranstehenden Ansprüche, **dadurch gekennzeichnet, daß** das Sensorelement im wesentlichen aus der eine Sensorfaser ausbildenden Glasfaser (7) besteht.

6. Implantatsystem nach Anspruch 5, **dadurch gekennzeichnet, daß** in die Sensorfaser mindestens ein als Bragg-Gitter wirkender Bereich (13, 14, 15) eingearbeitet ist.

7. Implantatsystem nach 5, **dadurch gekennzeichnet, daß** in die Sensorfaser eine durch die eingespeiste elektromagnetische Strahlung zur Fluoreszenz angeregte Substanz (17) eingebettet ist, deren Fluoreszenzeigenschaften unter Einwirkung der chemischen Umgebung außerhalb der Sensorfaser Änderungen erfahren.

8. Implantatsystem nach Anspruch 4 und 5, **dadurch gekennzeichnet, daß** die strahlungsreflektierende Beschichtung (18) aus einer Substanz besteht, die unter Einwirkung der chemischen Umgebung (19) außerhalb der Sensorfaser das Refilexionsverhalten für die elektromagnetische Strahlung in der Sensorfaser verändert.

9. Implantatsystem nach einem der Ansprüche 1 bis 3, **dadurch gekennzeichnet, daß** das Sensorelement die Glasfaser (7) umfaßt und ein weiteres Sensorglied (12), welches über die Glasfaser (7) mit der Meßeinrichtung (11) verbunden ist.

10. Implantatsystem nach Anspruch 9 **dadurch gekennzeichnet, daß** das Sensorglied (12) ein Drucksensor (25) mit einer flexiblen Membran (26) und einem von dieser bewegbaren Spiegelelement (27) ist, weiches die in die Glasfaser (7) eingespeiste elektromagnetische Strahlung je nach Stellung unterschiedlich reflektiert.

11. Implantatsystem nach Anspruch 9, **dadurch gekennzeichnet, daß** das Sensorglied (12) ein Fabry-Pérot-Interferometer ist.

12. Implantatsystem nach Anspruch 11, **dadurch gekennzeichnet, daß** das Fabry-Pérot-Interferometer als auf das Ende (20) der Glasfaser (7) aufkontaktiertes Dünnschicht-Interferometer (21, 22, 24) ausgebildet ist, dessen aktive Schicht (24) unter dem Einfluß der Umgebung Dimensionsänderungen erfährt.

13. Implantatsystem nach Anspruch 11, **dadurch gekennzeichnet, daß** das Fabry-Pérot-Interferometer zwei Glasfasern (7, 22) mit polierten Endflächen (20, 21) umfaßt, deren Abstand (B) durch Umgebungseinflüsse veränderbar ist.

14. Implantatsystem nach einem der voranstehenden Ansprüche, **dadurch gekennzeichnet, daß** die Glasfaser (7) des Sensorelementes direkt mit der Meßeinrichtung (11) verbunden ist.

15. Implantatsystem nach Anspruch 14, **dadurch gekennzeichnet, daß** die Meßeinrichtung ein in den Körper implantierbarer Mikrocontroller ist.

16. Implantatsystem nach einem Ansprüche 1 bis 13, **dadurch gekennzeichnet, daß** die Glasfaser (7) mit einem Übertrager (8) verbunden ist, der ohne körperliche Verbindung Signale mit der Meßeinrichtung (11) austauscht.

17. Implantatsystem nach Anspruch 16, **dadurch gekennzeichnet, daß** der Übertrager (8) in den Körper implantierbar ist.

18. Implantatsystem nach Anspruch 16 oder 17, **dadurch gekennzeichnet, daß** der Übertrager (8) ein Transponder ist.

19. Implantatsystem nach Anspruch 16 oder 17, **dadurch gekennzeichnet, daß** der Übertrager eine Lichtquelle ist, der ein Lichtempfänger zugeordnet ist.

20. Implantatsystem nach Anspruch 19, **dadurch gekennzeichnet, daß** die Lichtquelle elektromagnetische Strahlung im Bereich zwischen 650 und 1000 nm aussendet.

21. Implantatsystem nach einem der voranstehenden Ansprüche, **dadurch gekennzeichnet, daß** der Meßeinrichtung (11) ein Strahlungssender (29) zugeordnet ist, der über eine Glasfaser (7; 30) im Implantat (1) Strahlung in das Innere des Implantates (1) transportiert.

22. Implantatsystem nach Anspruch 21, **dadurch gekennzeichnet, daß** der Transport der Strahlung über die Glasfaser (7) eines Sensorelementes erfolgt.

23. Implantatsystem nach Anspruch 21, **dadurch gekennzeichnet, daß** der Transport der Strahlung über eine Glasfaser (30) erfolgt, die zusätzlich zu der Glasfaser (7) eines Sensorelementes in das Implantat (1) eingebettet ist.

24. Implantatsystem nach einem der Ansprüche 21 bis 23, **dadurch gekennzeichnet, daß** Wellenlänge und Intensität der transportierten Strahlung so gewählt sind, daß die Strahlung in dem Verbundwerkstoff des Implantates mechanische und/oder stoffliche Veränderungen hervorruft.

25. Implantatsystem nach einem der Ansprüche 21 bis 24, **dadurch gekennzeichnet, daß** der Meßeinrichtung (11) und dem Strahlungssender (29) eine Steuerung (31) zugeordnet ist, die den Strahlungssender in Abhängigkeit von den Meßgrößen der Meßeinrichtung (11) aktiviert.

## Claims

1. A medical implant system having an implant (1) made of a composite material, in which glass fibres (7) are embedded, wherein a sensor element embedded in the implant (1) and comprising at least one of the glass fibres (7) is connected to a measuring device (11) which measures a physical property of the sensor element or of its environment, or of a change therein, **characterised in that** the glass fibres (7) in the form of a tissue, or a piece of knitted material or a fleece, are embedded in the composite material as mechanical reinforcement.

2. An implant system according to Claim 1, **characterised in that** the glass fibres (7) in the composite material are distributed over the whole extent of the implant (1).

3. An implant system according to any one of the preceding claims, **characterised in that** the measuring device (11) supplies electromagnetic radiation to the sensor element and measures physical properties of the sensor element or of its environment from the nature of the radiation passing through and/or being reflected.

4. An implant system according to Claim 3, **characterised in that** the glass fibre (7) of the sensor element is provided with a radiation-reflecting coating (18).

5. An implant system according to any one of the preceding claims, **characterised in that** the sensor element consists substantially of the glass fibre (7) forming a sensor fibre.

6. An implant system according to Claim 5, **characterised in that** at least one region (13, 14, 15) functioning as a Bragg lattice is embedded in the sensor fibre.

7. An implant system according to [Claim] 5, **characterised in that** a substance (17) in which fluorescence is induced by the supplied electromagnetic radiation is embedded in the sensor fibre, the fluorescence properties of which substance undergo changes under the influence of the chemical environment outside the sensor fibre.

8. An implant system according to Claims 4 and 5, **characterised in that** the radiation-reflecting coating (18) consists of a substance which, under the influence of the chemical environment (19) outside the sensor fibre, alters the reflection behaviour in respect of the electromagnetic radiation within the sensor fibre.

9. An implant system according to any one of Claims 1 to 3, **characterised in that** the sensor element comprises the glass fibre (7) and another sensor member (12) which is connected to the measuring device (11) via the glass fibre (7).

10. An implant system according to Claim 9, **characterised in that** the sensor member (12) is a pressure sensor (25) having a flexible membrane (26) and a mirror element (27) movable by the said membrane, which mirror element position-dependently variously reflects the electromagnetic radiation supplied to the glass fibre (7).

11. An implant system according to Claim 9, **characterised in that** the sensor member (12) is a Fabry-Pérot interferometer.

12. An implant system according to Claim 11, **characterised in that** the Fabry-Pérot interferometer is in the form of a thin-layer interferometer (21, 22, 24) contacted on to the end (20) of the glass fibre (7), the active layer of which thin-layer interferometer undergoes changes in dimension under the influence of the environment.

13. An implant system according to Claim 11, **characterised in that** the Fabry-Pérot interferometer comprises two glass fibres (7, 22) having polished end faces (20, 21), the distance between which (B) is variable due to environmental influences.

14. An implant system according to any one of the preceding claims, **characterised in that** the glass fibre (7) of the sensor element is directly connected to the measuring device (11).

15. An implant system according to Claim 14, **characterised in that** the measuring device is a microcontroller implantable in the body.

16. An implant system according to any one of Claims 1 to 13, **characterised in that** the glass fibre (7) is connected to a transmitter (8) which, without physical contact, exchanges signals with the measuring device (11).

17. An implant system according to Claim 16, **characterised in that** the transmitter (8) is implantable in the body.

18. An implant system according to Claim 16 or 17, **characterised in that** the transmitter (8) is a transponder.

19. An implant system according to Claim 16 or 17, **characterised in that** the transmitter is a light source with which a light detector is associated.

20. An implant system according to Claim 19, **characterised in that** the light source emits electromagnetic radiation in the range between 650 and 1000 nm.

21. An implant system according to any one of the preceding claims, **characterised in that** the measuring device (11) is associated with a radiation transmitter (29) which, via a glass fibre (7; 30) in the implant (1), transports radiation into the interior of the implant (1).

22. An implant system according to Claim 21, **characterised in that** the radiation is transported via the glass fibre (7) of a sensor element.

23. An implant system according to Claim 21, **characterised in that** the radiation is transported via a glass fibre (30) which, like the glass fibre (7) of a sensor element, is embedded in the implant (1).

24. An implant system according to any one of Claims 21 to 23, **characterised in that** the wavelength and intensity of the transported radiation is selected such that the radiation induces mechanical and/or material changes in the composite material of the implant

25. An implant system according to any one of Claims 21 to 24, **characterised in that** the measuring device (11) and the radiation transmitter (29) are associated with a controller (31) which activates the radiation transmitter as a function of the measured variables of the measuring device (11).

## Revendications

1. Système d'implant médical avec un implant (1) en un matériau composite dans lequel des fibres de verre (7) sont incluses, où un élément capteur comprenant au moins l'une des fibres de verre (7) qui est inclus dans l'implant (1) est relié à un dispositif de mesure (11) qui détermine une propriété physique de l'élément capteur ou de son environnement ou une modification de celle-ci, **caractérisé en ce que** les fibres de verre (7) sont incluses dans le matériau composite à titre de renfort mécanique sous forme d'un tissu, d'un tissu à mailles ou d'un non-tissé.

2. Système d'implant selon la revendication 1 **caractérisé en ce que** les fibres de verre (7) sont réparties dans le matériau composite sur toute l'étendue de l'implant (1).

3. Système d'implant selon l'une des revendications précédentes **caractérisé en ce que** le dispositif de mesure (11) injecte un rayonnement électromagnétique dans l'élément capteur et détermine des propriétés physiques de l'élément capteur ou de son environnement d'après le type du rayonnement passant et/ou réfléchissant.

4. Système d'implant selon la revendication 3 **caractérisé en ce que** la fibre de verre (7) de l'élément capteur est munie d'un revêtement (18) réfléchissant les rayonnements.

5. Système d'implant selon l'une des revendications précédentes **caractérisé en ce que** l'élément capteur consiste essentiellement en la fibre de verre (7) formant une fibre capteur.

6. Système d'implant selon la revendication 5 **caractérisé en ce qu'**au moins un domaine (13, 14, 15) agissant à la manière d'un réseau de Bragg est formé dans la fibre capteur.

7. Système d'implant selon la revendication 5 **caractérisé en ce qu'**une substance (17) excitée à la fluorescence par le rayonnement électromagnétique injecté, dont les propriétés de fluorescence subissent des modifications sous l'effet de l'environnement chimique à l'extérieur de la fibre capteur, est incluse dans la fibre capteur.

8. Système d'implant selon les revendications 4 et 5 **caractérisé en ce que** le revêtement (18) réfléchissant les rayonnements consiste en une substance qui modifie le comportement de réflexion pour le rayonnement électromagnétique dans la fibre capteur sous l'effet de l'environnement chimique (19) à l'extérieur de la fibre capteur.

9. Système d'implant selon l'une des revendications 1 à 3 **caractérisé en ce que** l'élément capteur comprend la fibre de verre (7) et un autre membre capteur (12) qui est relié au dispositif de mesure (11) par la fibre de verre (7).

10. Système d'implant selon la revendication 9 **caractérisé en ce que** le membre capteur (12) est un capteur de pression (25) avec une membrane flexible (26) et un élément de miroir (27) déplaçable par celle-ci, qui réfléchit le rayonnement électromagnétique injecté dans la fibre de verre (7) de manière différente en fonction de la position.

11. Système d'implant selon la revendication 9 **caractérisé en ce que** le membre capteur (12) est un interféromètre de Fabry-Pérot.

12. Système d'implant selon la revendication 11 **caractérisé en ce que** l'interféromètre de Fabry-Pérot est sous forme d'un interféromètre à couche mince (21, 22, 24) appliqué par contact sur l'extrémité (20) de la fibre de verre (7), dont la couche active (24) subit des modifications de dimensions sous l'influence de l'environnement.

13. Système d'implant selon la revendication 11 **caractérisé en ce que** l'interféromètre de Fabry-Pérot comprend deux fibres de verre (7, 22) avec des surfaces terminales polies (20, 21) dont la distance (B) est modifiable par des influences de l'environnement.

14. Système d'implant selon l'une des revendications précédentes **caractérisé en ce que** la fibre de verre (7) de l'élément capteur est reliée directement au dispositif de mesure (11).

15. Système d'implant selon la revendication 14 **caractérisé en ce que** le dispositif de mesure est un microcontrôleur implantable dans le corps.

16. Système d'implant selon l'une des revendications 1 à 13 **caractérisé en ce que** la fibre de verre (7) est reliée à un transmetteur (8) qui échange des signaux avec le dispositif de mesure (11) sans liaison physique.

17. Système d'implant selon la revendication 16 **caractérisé en ce que** le transmetteur (8) est implantable dans le corps.

18. Système d'implant selon la revendication 16 ou 17 **caractérisé en ce que** le transmetteur (8) est un transpondeur.

19. Système d'implant selon la revendication 16 ou 17 **caractérisé en ce que** le transmetteur est une source lumineuse à laquelle est associé un récepteur de lumière.

20. Système d'implant selon la revendication 19 **caractérisé en ce que** la source lumineuse émet un rayonnement électromagnétique dans le domaine entre 650 et 1000 nm.

21. Système d'implant selon l'une des revendications précédentes **caractérisé en ce qu'**un émetteur de rayonnement (29) qui transporte un rayonnement à l'intérieur de l'implant (1) par le biais d'une fibre de verre (7 ; 30) dans l'implant (1) est associé au dispositif de mesure (11).

22. Système d'implant selon la revendication 21 **caractérisé en ce que** le transport du rayonnement a lieu par le biais de la fibre de verre (7) d'un élément capteur.

23. Système d'implant selon la revendication 21 **caractérisé en ce que** le transport du rayonnement a lieu par le biais d'une fibre de verre (30) qui est incluse dans l'implant (1) en plus de la fibre de verre (7) d'un élément capteur.

24. Système d'implant selon l'une des revendications 21 à 23 **caractérisé en ce que** la longueur d'onde et l'intensité du rayonnement transporté sont choisies de telle manière que le rayonnement provoque des modifications mécaniques et/ou matérielles dans le matériau composite de l'implant.

25. Système d'implant selon l'une des revendications 21 à 24 **caractérisé en ce qu'**une commande (31) qui active l'émetteur de rayonnement en fonction des grandeurs de mesure du dispositif de mesure (11) est associée au dispositif de mesure (11) et à l'émetteur de rayonnement (29).
